# EUROPÄISCHE PATENTSCHRIFT

(11) **EP 1 318 402 B1**
(45) Veröffentlichungstag und Bekanntmachung des Hinweises auf die Patenterteilung: **20.04.2005**
(21) Anmeldenummer: 01128848.7
(22) Anmeldetag: 04.12.2001
(51) Int. Cl.: G01N 33/68, G01N 33/569

(54) **Verfahren zur Diagnose von Sepsis unter Bestimmung von CA 125**
Diagnosis of sepsis determining CA 125
Diagnostic de la septicémie par détermination de la CA 125

(43) Veröffentlichungstag der Anmeldung: 11.06.2003
(73) Patentinhaber: B.R.A.H.M.S Aktiengesellschaft, 16761 Hennigsdorf (DE)
(72) Erfinder: Bergmann, Andreas, Dr., 12351 Berlin (DE)
(74) Vertreter: Andrae, Steffen, Dr.

(56) Entgegenhaltungen:
- PETÄJÄ J ET AL: "Serum Tumor Marker CA 125 Is an Early and Sensitive Indicator of Veno-Occlusive Disease in Children Undergoing Bone Marrow Transplantation" CLINICAL CANCER RESEARCH, Bd. 6, Februar 2000 (2000-02), Seiten 531-535, XP001076653
- MOLEY KELLE H ET AL: "Pelvic inflammatory disease: Correlation of severity with CA-125 levels." JOURNAL OF REPRODUCTIVE MEDICINE, Bd. 41, Nr. 5, 1996, Seiten 341-346, XP008003112 ISSN: 0024-7758
- ZEILLEMAKER A M ET AL: "CA 125 secretion by peritoneal mesothelial cells." JOURNAL OF CLINICAL PATHOLOGY (LONDON), Bd. 47, Nr. 3, 1994, Seiten 263-265, XP008003113 ISSN: 0021-9746
- REINHART, K ET AL: "Intensivmedizin: Sepsis und septischer Schock (Seiten 756-760)" 2001 , THIEME VERLAG XP008003130 * Seite 756, rechte Spalte, Absatz 3 * * das ganze Dokument *
- BEISHUIZEN A ET AL: "Endogenous mediators in sepsis and septic shock" ADVANCES IN CLINICAL CHEMISTRY, Bd. 33, 1999, Seiten 55-131, XP008003176

## Beschreibung

Die Erfindung betrifft ein neues Verfahren zur Sepsisdiagnose, bei dem oder im Rahmen dessen der an sich in der medizinischen Diagnostik als typischer Tumormarker bekannte Parameter CA 125 bestimmt wird.

Die Erfindung beruht auf dem erstmaligen Nachweis stark erhöhter Konzentrationen von CA 125 in Seren von Patienten, bei denen aufgrund klinischer Befunde und gleichzeitig erhöhten Serumkonzentrationen des bekannten Sepsismarkers Procalcitonin eine Sepsis diagnostiziert worden war.

Die vorliegende Erfindung hat ihren Ursprung in intensiven Forschungsarbeiten der Anmelderin im Zusammenhang mit weiteren Verbesserungen der Diagnose und Therapie von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismer wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Bei Sepsis bzw. dem septische Schock weiten sich entzündungsspezifische Reaktionskaskaden unkontrolliert auf den gesamten Körper aus und können dabei, im Sinne einer überschießenden systemischen Immunantwort, lebensbedrohlich werden. Zu den gegenwärtigen Kenntnissen über das Auftreten und die möglichen Rolle einzelner Gruppen endogener sepsisspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung wird daher der Begriff Sepsis in Anlehnung an die Definitionen verwendet, wie sie den genannten Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß oder vermutet, dass sie an einem Entzündungsgeschehen beteiligt sind, eignen sich dabei für Zwecke der klinischen Sepsisdiagnostik insbesondere solche, die bei Sepsis oder bestimmten Phasen einer Sepsis mit hoher Sensitivität und Spezifität auftreten bzw. deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen mit dem jeweiligen Biomarker im Vordergrund, ohne dass dessen Rolle in der komplexen Kaskade der am Sepsisgeschehen beteiligten endogenen Substanzen genau bekannt sein muss.

Eine bekannte, als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zur Früherkennung einer Sepsis und zur frühen Unterscheidung einer infektiös bedingten Sepsis von schweren Entzündungen, die auf anderen Ursachen beruhen, eignet. Besonders wertvoll ist die Procalcitoninbestimmung ferner zur therapiebegleitenden Verlaufsbeobachtung einer Sepsis. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig bei der Anmelderin intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik von septischen Erkrankungen zu liefern vermögen. So werden insbesondere weitere Biomarker für die Sepsisdiagnostik gesucht, deren Serum- bzw. Plasmaspiegel zwar regelmäßig erhöht sind, die bei ihrer Bestimmung jedoch nicht einfach die Ergebnisse der Procalcitoninbestimmung duplizieren, sondern zusätzliche Informationen liefern, und zwar insbesondere zum Stadium des Sepsisgeschehens, d.h. eine dem Sepsisverlauf eher zeitlich zuzuordnende Information, und/oder zum Ausgangs- oder Schwerpunktorgan eines septischen Geschehens, d.h. eine lokalisierende Information. Ziel ist letztlich die Selektion eines Satzes von Sepsisparametern, die bei Sepsispatienten bzw. potentiellen Sepsispatienten gleichzeitig bestimmt werden, z.B. unter Nutzung der sog. Chip-Technologie oder immunchromatographischer Verfahren ("Point of Care" oder POC-Bestimmungen), und dabei in ihrer Gesamtheit ein Informationsmuster liefern, das den Informationswert der Bestimmung nur eines einzelnen Parameters klar übertrifft.

Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Da die bei Sepsis erhöhten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Sepsisgeschehen einzugreifen, um zum Beispiel die bei Sepsis beobachtete systemische Ausbreitung der Entzündung möglichst frühzeitig zu stoppen. In diesem Sinne sind nachweislich am Sepsisgeschehen beteiligte endogene Substanzen auch als potentielle therapeutische Targets anzusehen.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin signifikant erhöht ist, sondern auch für andere Substanzen, die zu den Peptid-Prohormonen gerechnet werden können, signifikant erhöhte Konzentrationen beobachtet werden können. Es sind in diesem Zusammenhang zu nennen die Peptid-Prohormone pro-Enkephalin, pro-Gastrin-Releasing Peptid (proGRP), pro-Endothelin-1, pro-Brain-Natriuretic-Peptid (pro-BNP), pro-Atrial-Natriuretic-Peptid (pro-ANP), pro-Leptin, pro-Neuropeptid-Y, pro-Somatostatin, pro-Neuropeptid-YY, pro-Interleukin 6 oder pro-Interleukin-10. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird nunmehr ein Ergebnis eines anderen hypothetischen Ansatzes für die Suche nach weiteren für die Sepsisdiagnostik geeigneten Biomolekülen berichtet. Sie beruht auf den Ergebnissen von Messungen der physiologischen Konzentrationen von Biomarkern, die bisher als typische Tumormarker angesehen und daher klinisch im wesentlichen zu Zwecken der Tumordiagnostik bestimmt wurden, in biologischen Proben, insbesondere Serumproben, von Sepsispatienten, bei denen keinerlei klinische Befunde auf das Vorliegen von Tumoren hinwiesen.

Überraschenderweise zeigte sich, dass bei Sepsis auch einige bisher als typische Tumormarker angesehene Biomoleküle signifikant erhöht sind. Das deutet darauf hin, dass diese nicht tumorspezifisch gebildet werden, sondern eher ein physiologisches Krisengeschehen anzeigen, das auch Gewebe bzw. Organe erfaßt, die diese Tumormarker freisetzen. Und obwohl, wie in dieser Anmeldung und gleichzeitig eingereichten weiteren Anmeldungen gezeigt wird, die Konzentrationen der fraglichen Biomoleküle bei Sepsis mit hoher Sensitivität erhöht sind, besteht gleichzeitig keine Korrelation der gemessenen Werte zu den ebenfalls signifikant erhöhten Procalcitoninkonzentrationen, d.h. bei einzelnen Patienten werden zwar beide Parameter erhöht gefunden, jedoch teilweise in ganz unterschiedlicher Ausprägung.

Die vorliegende Erfindung beruht auf dem erstmaligen Nachweis, dass bei Sepsis signifikant erhöhte physiologische Konzentrationen CA 125 gefunden werden, was diesen Parameter, insbesondere in Kombination mit der Bestimmung weiterer Sepsisparameter, für die differentielle Sepsisdiagnostik geeignet macht.

Das erfindungsgemäße Verfahren und bestimmte bevorzugte Ausgestaltungen davon sind in den Ansprüchen 1 bis 6 genauer definiert.

Es ist bisher nicht bekannt, dass die Konzentrationen von CA 125 in biologischen Flüssigkeiten, insbesondere die Serumkonzentrationen, bei Sepsis signifikant erhöht sind und dass daher eine Bestimmung der Konzentration von CA 125 auch für die Sepsisdiagnostik von Bedeutung sein könnte.

Aufgrund der vorliegenden Erfindung ist es möglich, die Bestimmung von CA 125 auch im Rahmen eines diagnostischen Sepsis-Nachweisverfahrens zu nutzen. Von besonderem Interesse ist die Eignung von CA 125 als Prognosemarker und Marker für die Überwachung der Krankheitsverlaufs von Sepsis, insbesondere im Rahmen einer Kombinationsmessung mit anderen Markern.

Neben einer Kombination mit einer Procalcitoninmessung kommt insbesondere eine Kombination des Messung von CA 125 mit der Bestimmung anderer Marker für Sepsis und systemische Entzündungen, die bisher als typische Tumormarker angesehen wurden, in Betracht, und zwar insbesondere mit CA 19-9, S100B oder an der Regulierung von Entzündungen beteiligten S100A-Proteinen, oder mit der Bestimmung der in den älteren, nachfolgend genannten deutschen Patentanmeldungen der Anmelderin beschriebenen neuartigen Sepsismarker Inflammin (DE 101 19 804.3) und CHP (DE 101 31 922.3), und/oder mit der Bestimmung von löslichen Cytokeratinfragmenten, insbesondere der neu aufgefundenen löslichen Cytokeratin-1-Fragmente (sCY1F;; DE 101 30 985.6) sowie der bekannten Tumormarker CYFRA-21 oder TPS und/oder eines oder mehrerer der o.g. Prohormone. Auch eine gleichzeitige Bestimmung des bekannten Entzündungsparameters C-reaktives Protein (CRP) kann vorgesehen sein. Aufgrund der in dieser und den parallelen Anmeldungen beschriebenen neuen Ergebnisse ist für die Sepsis-Feindiagnose außerdem generell eine Kombination mit Messungen von bekannten oder noch aufzufindenden Biomolekülen in Betracht zu ziehen, die als gewebe- bzw. organspezifische Entzündungsmarker geeignet sind.

Der Inhalt der genannten älteren Anmeldungen der Anmelderin ist durch die ausdrückliche Bezugnahme auf diese Anmeldungen als Teil der Offenbarung der vorliegenden Anmeldung anzusehen.

Als CA 125 (cancer antigen 125) wird ein Glykoprotein mit einer molaren Masse von ca. 200 kD und einem hohen Kohlenhydratanteil (ca. 25%) bezeichnet, das, wie in Bast et al., 1981, J.Clin.Invest. 68, (1981) pp.1331-1337 beschrieben wird, durch Reaktion mit einem bestimmten spezifischen monoklonalen Antikörper (OC-125) identifiziert wird. Es spielt eine adjuvante Rolle bei der Diagnostik, Therapieund Verlaufskontrolle des Ovarialkarzinoms und kann auch als zweiter Marker bei der Diagnose des Pankreaskarzinoms mitverwendet werden (vgl. Lothar Thomas (Hrsg.): Labor und Diagnose, Kapitel 34.4, S.969-973, 5.Auflage, 1998 TH-Books Verlagsgesellschaft).

Es ist bekannt, dass auch bei einer gewissen Zahl von Patienten mit nicht-malignen entzündlichen Erkrankungen wie Nierenbeckenentzündungen (Pyelitis, PID), Tuben- bzw. Eileiterentzündungen (Salpingitis), Bauchfellentzündungen (Peritonitis) und Tuberkulose erhöhte Spiegel von CA 125 gemessen werden können (vgl. z.B. J. Paavonen et al., (1989), Br J Obstet Gynaecol, 96:574-579; M.M.Pannekeet et al., (1995), Peritoneal Dialysis International, Vol.15, pp.217-225; C.I.Macri, Gynecol Obstet Invest 1994, 37:143-144; J. Fernandez B. de Quiros at al., The International Journal of Biological Markers, Vol. 10, No.3, 180-181; J. Petäjä et al., Clin.Canc.Res, Vol.6, 531-535 (2000); E. Moore et al., Infect.Dis.Obstet.Gynecol. 6:182-185, 1998).

Die Arbeit von J. Petäjä et al., Clin.Canc.Res, Vol.6, 531-535 (2000) beschreibt die Ergebnisse von Messungen des Parameters CA 125 im Serum von kindlichen Patienten, bei denen Knochenmarkstransplantationen (bone marrow transplantation; BMT) vorgenommen worden waren. Dabei wurde festgestellt, dass dieser Parameter bei 86% derjenigen Patienten, die nach der BMT als Komplikation eine Venenverschluß-Krankheit entwickelten (Veno-occlusive disease; VOD), um wenigstens 57% gegenüber dem vor der BMT gemessenen Wert anstieg, und zwar bevor die VOD aufgrund anderer Kriterien diagnostiziert wurde.

Lediglich zur systematischen Kontrolle wurden die bei den gleichen Patienten während des Meßzeitraums erhaltenen Meßwerte für den Parameter CA 125 außerdem noch unter dem Gesichtspunkt, ob zum Messzeitpunkt bei dem Patienten eine akute Infektion (als Sepsis bezeichnet) diagnostiziert war oder nicht, zwei Messwert-Gruppen zugeordnet. Es ergab sich bei dieser Art der Auswertung, dass das Vorliegen einer Infektion keinen erkennbaren zusätzlichen systematischen Einfluß auf die CA 125-Meßwerte hatte.

Da alle Messwerte, die den Gruppen "Sepsis/keine Sepsis" zugeordnet wurden, Messwerte von BMP-Patienten waren, bei denen die Werte für CA 125 z.T. aus anderen Gründen stark erhöht waren (bei den VOD-Patienten), kann aus den Ergebnissen in der genannten Veröffentlichung keinerlei logische Schlussfolgerung bezüglich einer Eignung von CA 125 als Marker im Rahmen der Sepsis-Differentialdiagnostik gezogen werden. Bei septischen Patienten wurden nach diesseitiger Kenntnis noch keine systematischen CA 125-Messungen durchgeführt bzw. über in der großen Mehrzahl der Fälle signifikant erhöhte Messwerten des typischen Tumormarkers CA 125 bei Patienten mit Sepsis wurde noch nichts bekannt.

Eine deutliche Erhöhung der CA 125-Konzentrationen bei der überwiegenden Zahl von Sepsispatienten wurde erstmals bei den Bestimmungen festgestellt, die im nachfolgenden Versuchsbericht unter Bezugnahme auf zwei Figuren beschrieben werden.

In den Figuren zeigen:
- Fig.1: die Ergebnisse der Bestimmung von CA 125 in den Seren von 171 Sepsispatienten im Vergleich mit einer Gruppe von 50 Kontrollpersonen (Blutspendern) ; und
- Fig.2: die Korrelation der Ergebnisse der CA 125-Bestimmungen der 171 Sepsispatienten von Fig.1 mit den Ergebnissen der Procalcitoninbestimmung.

### Versuchsbericht:

In 171 Seren von Sepsis-Patienten, bei denen hohe Werte für den Sepsismarker Procalcitonin (PCT) gefunden worden waren, wurden unter Verwendung eines kommerziellen Assays zur Bestimmung von CA 125 (KRYPTOR-CA125 II der B.R.A.H.M.S Diagnostica GmbH) die Serumkonzentrationen des Tumormarkers CA 125 bestimmt. Bei 68 % der Seren wurden stark erhöhte CA 125 Konzentrationen (mehr als 32 U/ml) gefunden.

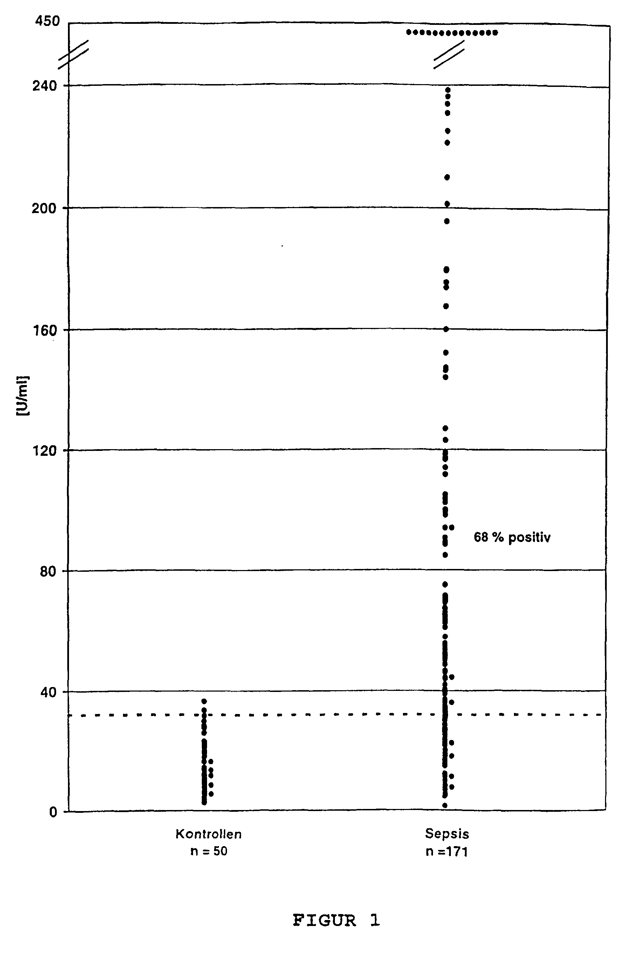

Eine graphische Darstellung der Messergebnisse ist in Figur 1 gezeigt.

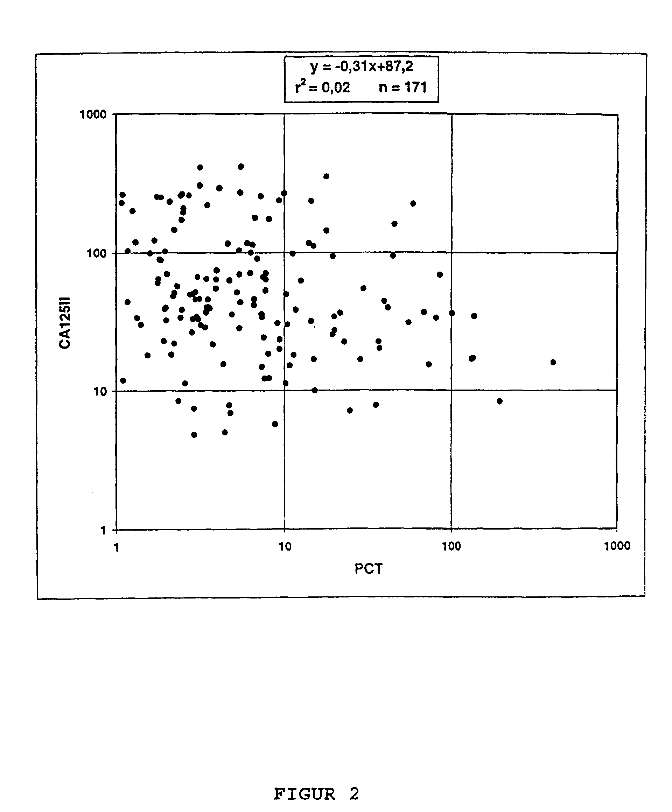

Werden die für individuelle Seren gemessenen Werte für CA 125 den für PCT gemessenen Werten gegenübergestellt, ergibt sich keine positive quantitative Korrelation in dem Sinne, dass in Seren mit hohen PCT Konzentrationen auch die höchsten CA 125-Werte gefunden werden. Figur 2 zeigt die bei einer solchen Gegenüberstellung gefundenen Korrelationen. Es ist zu erkennen, dass hohe CA 125-Werte (oberes Drittel des Diagramms) auch bei mäßigen PCT-Konzentrationen erhalten werden, und bei sehr hohen PCT-Konzentrationen (rechtes Drittel des Diagramms) mäßige Werte für CA 125.

Die weitgehende Unabhängigkeit der Ergebnisse der CA 125 Bestimmung von denen der PCT-Bestimmung zeigt, dass trotz der für beide Parameter bei Sepsis erhöhten Werte bei Sepsis unterschiedliche Effekte erfasst werden, was bedeutet, dass die Messung beider Parameter mehr Informationen liefert als die Messung nur eines der Parameter.

Die Kombination der Bestimmung von CA 125 mit der eines oder mehrerer anderer Sepsismarker bietet sich daher zur Verbesserung der Feindiagnostik von Sepsis und zur Verbesserung der Prognose des Krankheitsverlaufs und zur therapiebegleitenden Verlaufskontrolle bei Sepsispatienten an, wobei die klare Hoffnung besteht, dass die Interpretation der Ergebnisse solcher kombinatorischer Bestimmungen auf der Basis der genauen Auswertung einzelner möglichst vollständig dokumentierter Fälle (mit z.B. Angaben zur Art der Infektion, Grund und Verlauf der Sepsiserkrankung, Kenndaten zum Alter und Geschlecht der Patienten) mit der Anzahl der ausgewerteten Fälle stetig verbessert wird.

Die Bestimmung von CA 125 kann dabei nach irgendeinem beliebigen geeigneten Nachweisverfahren erfolgen, wobei jedoch die Bestimmung in einer Körperflüssigkeit eines Patienten auf immundiagnostischem Wege unter Verwendung geeigneter selektiver Antikörper unter praktischen Gesichtspunkten am vorteilhaftesten erscheint. Es stehen bereits kommerzielle Assays zur Bestimmung von CA 125 zur Verfügung, die auch im Rahmen der vorliegenden Erfindung genutzt werden können. Dabei ist gegebenenfalls auf eine gute Meßgenauigkeit im für die Sepsisdiagnostik relevanten Messbereich zu achten.

Somit kann die Bestimmung von CA 125 zur differentialdiagnostischen Früherkennung und zur Erkennung sowie für die Erstellung einer Verlaufsprognose, zur Beurteilung des Schweregrads und zur therapiebegleitenden Verlaufsbeurteilung von Sepsis erfolgen, indem man bei einem derartigen Verfahren in einer Probe einer biologischen Flüssigkeit eines Patienten den Gehalt von CA 125 bestimmt und aus der festgestellten Anwesenheit und/oder Menge von CA 125 auf das Vorliegen einer Sepsis schließt und das erhaltene Ergebnis mit dem Schweregrad, dem Fortschritt der Sepsis und/oder dem am stärksten von der Sepsis betroffenen Gewebe oder Organ korreliert und die Behandlungsmöglichkeiten entsprechend wählt und/oder die Behandlungsaussichten abschätzt.

## Patentansprüche

1. *In vitro*-Verfahren zur differentialdiagnostischen Früherkennung und Erkennung, für die Verlaufsprognose und die Beurteilung des Schweregrads und zur therapiebegleitenden Verlaufsbeurteilung von Sepsis, **dadurch gekennzeichnet, dass** man die Menge von CA 125 in einer biologischen Flüssigkeit eines Patienten, bei dem eine Sepsis vorliegt oder ein Verdacht auf Sepsis besteht, bestimmt und aus der bestimmten Menge an CA 125 Schlüsse hinsichtlich des Vorliegens, des zu erwartenden Verlaufs, des Schweregrads und/oder des Erfolgs eingeleiteter Maßnahmen zur Therapie der Sepsis zieht.

2. Verfahren nach Anspruch 1, **dadurch gekennzeichnet, dass** die Bestimmung von CA 125 mittels eines immundiagnostischen Bestimmungsverfahrens erfolgt.

3. Verfahren nach Anspruch 1 oder Anspruch 2, **dadurch gekennzeichnet, dass** es im Rahmen einer Multiparameter-Bestimmung durchgeführt wird, bei der gleichzeitig mindestens ein weiterer Sepsisparameter bestimmt wird und ein Messergebnis in Form eines Satzes von mindestens zwei Messgrößen gewonnen wird, der zur Sepsis-Feindiagnostik ausgewertet wird.

4. Verfahren nach Anspruch 3, **dadurch gekennzeichnet, dass** im Rahmen der Multiparameter-Bestimmung neben CA 125 wenigstens ein weiterer Parameter bestimmt wird, der ausgewählt ist aus der Gruppe, die besteht aus Procalcitonin, CA 19-9, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen und dem C-reaktiven Protein (CRP).

5. Verfahren nach Anspruch 3 oder 4, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

6. Verfahren nach Anspruch 5, **dadurch gekennzeichnet, dass** die Auswertung des mit der Messvorrichtung gewonnenen komplexen Messergebnisses mit Hilfe eines Computerprogramms erfolgt.

## Claims

1. *In vitro* method for early differential diagnosis and detection, for prognosis and assessment of the severity and therapy-accompanying assessment of the course of sepsis and sepsis-like systemic infections, **characterized in that** the amount of CA 125 is determined in a biological fluid of a patient in whom a sepsis is present or sepsis is suspected and conclusions are drawn from the determined amount of CA 125 with regard to the presence, the expected course, the severity and/or the success of initiated measures for the treatment of the sepsis.

2. Method according to Claim 1, **characterized in that** the determination of CA 125 is carried out by means of an immunodiagnostic assay method.

3. Method according to Claim 1 or Claim 2, **characterized in that** it is carried out in the course of a multiparameter determination in which at the same time at least one further sepsis parameter is determined, and a measured result in the form of a set of at least two measured variables, which is evaluated for fine sepsis diagnosis, is obtained.

4. Method according to Claim 3, **characterized in that**, in addition to CA 125, at least one further parameter which is selected from the group consisting of procalcitonin, CA 19-9, S100B, S100A proteins, soluble cytokeratin fragments, in particular CYFRA 21, TPS and/or soluble cytokeratin-1 fragments (sCY1F), the peptides inflammin and CHP, peptide prohormones and the C-reactive protein (CRP) is determined in the course of the multiparameter determination.

5. Method according to Claim 3 or 4, **characterized in that** the multiparameter determination is carried out as a simultaneous determination by means of a measuring apparatus operating according to the chip technology method or an immunochromatographic measuring apparatus.

6. Method according to Claim 5, **characterized in that** the evaluation of the complex measured result obtained using the measuring apparatus is carried out with the aid of a computer program.

## Revendications

1. Procédé *in vitro* de reconnaissance précoce et de reconnaissance diagnostique différentielle, pour le pronostic d'évolution et l'évaluation du taux de sévérité et pour l'évaluation d'évolution accompagnant une thérapie de la septicémie, **caractérisé en ce que** l'on détermine la quantité de CA 125 dans un liquide biologique d'un patient chez lequel une septicémie est présente ou il existe un soupçon de septicémie et à partir de la quantité déterminée en CA 125, on conclut de la présence, de l'évolution à attendre, du taux de sévérité et/ou de la suite des mesures à prendre pour la thérapie de la septicémie.

2. Procédé selon la revendication 1, **caractérisé en ce que** la détermination du CA 125 est réalisée à l'aide d'un procédé de détermination immunodiagnostique.

3. Procédé selon la revendication 1 ou 2, **caractérisé en ce qu'**il est réalisé dans le cadre d'une détermination de plusieurs paramètres, dans laquelle on détermine simultanément, au moins un autre paramètre de la septicémie et l'on obtient un résultat de mesure sous forme d'un jeu d'au moins deux mesures, qui est évalué pour le diagnostic fin de la septicémie.

4. Procédé selon la revendication 3, **caractérisé en ce que** dans le cadre de la détermination de plusieurs paramètres, on détermine en plus du CA 125, au moins un autre paramètre, qui est choisi dans le groupe qui consiste en la procalcitonine, le CA 19-9, le S100B, les protéines S100A, des fragments solubles de cytokératine, en particulier le CYFRA 21, le TPS et/ou des fragments solubles de la cytokératine-1 (sCY1F), et les peptides inflammine et CHP, des prohormones peptidiques et la protéine C réactive (CRP).

5. Procédé selon la revendication 3 ou 4, **caractérisé en ce que** la détermination de plusieurs paramètres est réalisée comme une détermination simultanée à l'aide d'un dispositif de mesure par technologie à puce ou d'un dispositif de mesure immunochromatographique.

6. Procédé selon la revendication 5, **caractérisé en ce que** l'évaluation des résultats de mesure complexes obtenus avec le dispositif de mesure est réalisée à l'aide d'un programme d'ordinateur.
